# DEMANDE DE BREVET EUROPEEN

(11) **EP 1 417 957 A1**
(43) Date de publication de la demande: **12.05.2004**
(21) Numéro de dépôt: 03292550.5
(22) Date de dépôt: 14.10.2003
(51) Int. Cl.: A61K 7/48

(54) **Composition cosmétique contenant des fibres et un polymère associatif**

(30) Priorité: 06.11.2002 FR 0213875
(71) Demandeur: L'OREAL, 75008 Paris (FR)
(72) Inventeur: Chevalier, Véronique, 94440 Villecresnes (FR); Collette Annick, 94100 St Maur des Fosses (FR)
(74) Mandataire: Rasson, Catherine

(57) **Abrégé**

L'invention se rapporte à une composition pour application topique comprenant dans un milieu physiologiquement acceptable, des fibres et un polyuréthanne associatif, le milieu comprenant une phase aqueuse et une phase huileuse comprenant au moins une huile.

La composition selon l'invention peut constituer notamment une composition de soin ou de maquillage des matières kératiniques telles que la peau, y compris le cuir chevelu, les lèvres du visage et les phanères comme les cils, les sourcils, les ongles et les cheveux.

L'invention a aussi pour objet l'utilisation d'un polyuréthanne associatif, dans une composition contenant des fibres, pour obtenir une composition homogène et lisse.

## Description

La présente invention se rapporte à une composition pour application topique sous forme d'émulsion, contenant des fibres et un polyuréthanne associatif, et à ses utilisations notamment dans les domaines cosmétique et dermatologique, en particulier pour le soin, le traitement ou le maquillage des matières kératiniques telles que la peau, y compris le cuir chevelu, les lèvres du visage et les phanères comme les cils, les sourcils, les ongles et les cheveux. La présente invention se rapporte aussi à l'utilisation d'un polyuréthanne associatif, dans une composition contenant des fibres, pour obtenir une composition homogène et lisse, même après un certain temps d'utilisation.

Il est connu d'incorporer des fibres dans les compositions cosmétiques. Ainsi, le document JP07-196440 décrit des compositions cosmétiques contenant des fibres de polyamide courtes, celles-ci donnant aux dites compositions un toucher velouté et une bonne tenue cosmétique. Par ailleurs, dans le domaine du maquillage de la peau, il est connu d'utiliser des fibres dans des produits de maquillage notamment pour leurs effets allongeant dans des mascaras (voir JP-A-57/158714), leur toucher « textile» (voir JP-A-7/196440), leur effet de tissus ou encore leurs propriétés hydratantes dans des rouges à lèvres (voir le document US-A-5 498 407) ou pour améliorer les contours du rouge à lèvres sur les bords des lèvres (voir le document EP-A-0 106 762).

Ces fibres peuvent être notamment incorporées dans des émulsions, soit eau-dans-huile (E/H) soit huile-dans-eau (H/E), comme décrit par exemple dans les documents EP-A-1,090,626, EP-A-1,090,627, EP-A-1,092,424, EP-A-1,092,425.

Malheureusement, au cours du temps, surtout quand les fibres sont en forte proportion, par exemple en une proportion supérieure à 5 % en poids, et notamment quand les compositions sont sous forme d'émulsions, les compositions contenant des fibres développent un aspect filandreux lors de leur utilisation, et elles deviennent de ce fait moins agréables à voir et à utiliser.

Il subsiste donc le besoin d'une composition pour application topique, et notamment sous forme d'émulsion, contenant des fibres et ne présentant pas les inconvénients ci-dessus, c'est-à-dire restant lisse et homogène tout au long du temps d'utilisation par la consommatrice.

La demanderesse a trouvé de manière surprenante que l'utilisation d'un polyuréthanne associatif permettait l'obtention d'une composition ayant une très bonne stabilité et de bonnes propriétés cosmétiques.

L'invention s'applique non seulement aux produits de soin, de traitement et/ou de maquillage de la peau y compris du cuir chevelu, aussi bien du visage que du corps humain, et des lèvres du visage, mais aussi aux produits de maquillage des phanères comme les cils, les sourcils et les ongles, et aux produits de soin et/ou de traitement des cheveux.

De façon plus précise, l'invention a pour objet une composition pour application topique comprenant dans un milieu physiologiquement acceptable, des fibres et au moins un polyuréthanne associatif, le milieu comprenant une phase aqueuse et une phase huileuse comprenant au moins une huile.

On entend ici par « application topique » une application externe sur les matières kératiniques, que sont notamment la peau, le cuir chevelu, les cils, les sourcils, les ongles, les muqueuses et les cheveux.

On entend par « milieu physiologiquement acceptable » un milieu compatible avec la peau, les lèvres, les ongles, le cuir chevelu et/ou les cheveux.

La composition obtenue présente une agréable douceur lors de son application sur les matières kératiniques et notamment sur la peau, et elle présente une bonne tenue cosmétique. En outre, grâce au polyuréthanne associatif, on obtient une composition qui reste homogène et stable dans le temps, et ne prend pas un aspect filandreux. En outre, cette composition a l'avantage d'être "auto-lissante", c'est-à-dire qu'après chaque utilisation, il se reforme une surface lisse et homogène à la surface du pot, comme si le pot était neuf, et que lorsque la crème est prise avec un doigt ou tout autre moyen (éponge, spatule), la trace de ce doigt ou de ce moyen ne subsiste absolument pas.

Aussi, l'invention a encore pour objet l'utilisation cosmétique d'un polyuréthanne associatif, dans une composition pour application topique, contenant des fibres, pour obtenir une composition constamment lisse et homogène.

On entend par "constamment lisse et homogène" une composition qui ne prend pas un aspect filandreux au cours du temps et dont la surface redevient lisse après utilisation.

La composition de l'invention a généralement l'aspect d'une crème, c'est-à-dire qu'elle présente généralement une viscosité allant de 10 à 300 poises (soit 1 à 30 Pa.s), de préférence de 30 à 250 poises (soit 3 à 25 Pa.s) et mieux de 75 à 250 poises (soit 7,5 à 25 Pa.s), mesurée à 25°C avec un viscosimètre Rhéomat 180.

### Fibres

Par "fibre", il faut comprendre dans la présente demande, un objet de longueur L et de diamètre D, tel que L soit très supérieur à D, D étant le diamètre du cercle dans lequel s'inscrit la section de la fibre. En particulier, le rapport L/D (ou facteur de forme) est choisi dans la gamme allant de 3,5 à 2 500, de préférence de 5 à 500 et mieux de 5 à 150.

Les fibres peuvent être présentes dans la composition selon l'invention en une quantité allant de 0,5 à 50 % en poids, de préférence de 5 à 40 % en poids, mieux 5 à 30 % en poids et encore mieux de 10 à 20 % en poids par rapport au poids total de la composition.

Les fibres utilisables dans la composition de l'invention peuvent être des fibres d'origine synthétique ou naturelle, minérale ou organique. Elles peuvent être courtes ou longues, unitaires ou organisées par exemple tressées. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres ont une longueur allant de 1 nm à 20 mm, de préférence de 10 nm à 5 mm et mieux de 0,1 mm à 1,5 mm. Leur section peut être comprise dans un cercle de diamètre allant de 2 nm à 100 µm, de préférence allant de 20 nm à 20 µm et mieux de 5 µm à 50 µm. Par ailleurs, elles sont caractérisées par leur poids, souvent donné en denier ou décitex.

Les fibres peuvent être celles utilisées dans la fabrication des textiles et notamment des fibres de soie, de coton, de laine, de lin, de cellulose extraites notamment du bois, des légumes ou des algues, de polyamide (Nylon®, notamment sous les appellations Nylon 6 = Polyamide 6 ; Nylon 6,6 = Polyamide 66) ; de rayonne, de viscose, d'acétate notamment d'acétate de rayonne, d'acétate de cellulose ou d'acétate de soie, de poly-p-phénylène téréphtalamide notamment de Kevlar®, en acrylique notamment de polyméthacrylate de méthyle ou de poly-2-hydroxyéthylméthacrylate, de polyoléfine et notamment de polyéthylène ou de polypropylène, de verre, de silice, d'aramide, de carbone notamment sous forme graphite, de Téflon®, de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-avant, comme des fibres de polyamide/polyester, les fibres chirurgicales, et les mélanges de ces fibres.

On peut utiliser comme fibres chirurgicales, les fibres synthétiques résorbables préparées à partir d'acide glycolique et de caprolactone (« Monocryl » de la société Johnson & Johnson) ; les fibres synthétiques résorbables du type copolymère d'acide lactique et d'acide glycolique (« Vicryl » de la société Johnson & Johnson) ; les fibres de polyester téréphtalique (« Ethibond » de la société Johnson & Johnson) et les fils d'acier inoxydable (« Acier » de la société Johnson & Johnson).

Par ailleurs, les fibres peuvent être traités ou non en surface, enrobées ou non. Comme fibres enrobées utilisables dans l'invention, on peut citer des fibres de polyamide enrobées de sulfure de cuivre pour un effet antistatique (par exemple les fibres R-STAT de la société Rhodia) ou un autre polymère permettant une organisation particulière des fibres (traitement de surface spécifique) ou traitement de surface induisant des effets de couleurs/hologrammes (fibre « Lurex » de la société Sildorex, par exemple).

Les fibres utilisables dans la composition selon l'invention sont de préférence des fibres souples, et la composition est de préférence exempte de fibres rigides.

Selon un mode préféré de réalisation de l'invention, les fibres utilisables dans la composition selon l'invention sont des fibres de polyamide ou de poly-p-phénylène téréphtalamide. Leur longueur peut aller de 0,1 à 5 mm, de préférence de 0,25 à 1,6 mm, et leur diamètre moyen peut aller de 5 à 50 µm.

Selon un mode préféré de réalisation de l'invention, les fibres sont choisies parmi les fibres de Nylon 6 (ou Polyamide 6) (nom INCI : Nylon 6), de Nylon 6,6 (ou Polyamide 66) (Nom INCI : Nylon 66), et leurs mélanges.

En particulier, on peut utiliser les fibres de polyamide commercialisées par les Etablissements P. Bonte sous le nom Polyamide 0.9 Dtex 0.3 mm (nom INCI : Nylon 66), ayant un diamètre moyen de 6 µm, un poids d'environ 0.9 dtex et une longueur allant de 0,3 mm à 1,5 mm. On peut aussi utiliser les fibres de poly-p-phénylène téréphtalamide de diamètre moyen de 12 µm et de longueur d'environ 1,5 mm comme celles vendues sous le nom de Kevlar Floc par la société Du Pont Fibres.

### Polyuréthanne associatif

Les polyuréthannes associatifs sont des copolymères séquences non ioniques comportant dans la chaîne, à la fois des séquences hydrophiles de nature le plus souvent polyoxyéthylénée et des séquences hydrophobes (lipophiles) qui peuvent être des enchaînements aliphatiques seuls et/ou des enchaînements cycloaliphatiques et/ou aromatiques.

En particulier, ces copolymères comportent au moins deux chaînes lipophiles hydrocarbonées, ayant de 6 à 30 atomes de carbone (C₆ à C₃₀), séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter une chaîne hydrocarbonée à un bout ou aux deux bouts d'une séquence hydrophile.

Les polymères peuvent être séquences sous forme de tribloc ou multibloc. Les séquences hydrophobes peuvent donc être à chaque extrémité de la chaîne (par exemple : copolymère tribloc à séquence centrale hydrophile) ou réparties à la fois aux extrémités et dans la chaîne (copolymère multiséquencé par exemple). Les polymères peuvent être également en greffons ou en étoile.

La séquence hydrophile est de préférence une chaîne oxyalkylénée, en particulier une chaîne oxyéthylénée.

Ainsi, de préférence, les polymères présentent une chaîne hydrophile oxyéthylénée. En outre, ce sont de préférence des polymères triblocs, notamment des polymères triblocs dont la séquence hydrophile est une chaîne oxyéthylénée, comportant de 50 à 1 000 groupements oxyéthylénés, et de préférence de 70 à 500 groupements oxyéthylénés.

En général, les polyuréthannes associatifs comportent une liaison uréthanne entre les séquences hydrophiles, d'où l'origine du nom. Par extension, figurent aussi parmi les polyuréthannes associatifs, des polymères dont les séquence hydrophiles sont liées par d'autres liaisons chimiques aux séquences lipophiles. Toutefois, selon un mode préféré de réalisation de l'invention, le polyuréthanne associatif utilisé contient une liaison uréthanne. Ces polyuréthannes peuvent être utilisés sous forme pure ou en solution ou dispersion dans de l'eau ou dans des milieux hydroalcooliques.

A titre d'exemple de polymères associatifs utilisables dans l'invention, on peut citer le polymère C₁₆-OE₁₃₆-C₁₆, OE étant un motif oxyéthyléné, vendu par la société HULS sous le nom SER-AD FX1100, qui est une molécule à fonction uréthanne, de poids moléculaire moyen en poids de 30000 (nom INCI : Steareth-100/PEG-136/HMDI Copolymer). Comme polymère associatif, on peut aussi utiliser aussi les polyuréthannes à groupements polyéther et urée, commercialisés sous les noms de Rheolate 205, Rheolate 204 et Rheolate 208 par la société RHEOX (nom INCI : Polyether-urea-polyurethane). Ces polyuréthannes associatifs sont vendus sous forme pure.

Comme solutions ou dispersions de ces polymères, on peut citer par exemple les polyuréthannes commercialisés sous les dénominations SER-AD FX1010 et le SER-AD 1035 par la société HÜLS (nom INCI : Polyurethane), ceux commercialisés sous les dénominations Rheolate 255, Rheolate 278 et Rheolate 244 par la société RHEOX (nom INCI : Polyether-urea-polyurethane), ceux commercialisés sous les dénominations DW 1206F, DW 1206J, DW 1206B, DW 1206G par la société RÖHM & HAAS (nom INCI : Polyurethane), et celui commercialisé sous la dénomination Acrysol RM 2020 de la société RÖHM & HAAS.

Les polymères utilisables dans l'invention sont en particulier ceux décrits dans l'article de G. Fonnum, J. Bakke et Fk. Hansen - Colloid Polym. Sci 271, 380.389 (1993).

Selon un mode préféré de réalisation de l'invention, on utilise un polyuréthanne soluble dans l'eau car une solution de polyuréthanne est mieux appropriée au but de l'invention qu'une dispersion.

La composition selon l'invention contient un ou plusieurs polyuréthannes associatifs, en une quantité suffisante pour obtenir le but recherché, c'est-à-dire obtenir une composition qui reste lisse, ne prenne pas un aspect filandreux au cours du temps d'utilisation par la consommatrice et qui soit auto-lissante.

La quantité de polyuréthanne(s) associatif(s) en matière active peut aller par exemple de 0,5 à 10 % en poids, de préférence de 1 à 5 % en poids et mieux de 1,5 à 3 % en poids par rapport au poids total de la composition.

La composition selon l'invention comporte une phase huileuse et une phase aqueuse. Elle peut être à phase continue ou externe huileuse (E/H) ou à phase continue aqueuse (H/E), et elle peut être une émulsion simple (H/E ou E/H) ou une émulsion triple (E/H/E ou H/E/H) ou multiple. Elle se présente généralement en particulier sous la forme d'une émulsion H/E constituant une crème ou une pommade.

Lorsque l'émulsion est du type huile-dans-eau, le polyuréthanne associatif permet l'obtention d'un « réseau » polymérique sur la peau, lors de l'application, assurant notamment l'autocicatrisation et le nivellement du relief de la peau.

Selon un mode préféré de réalisation de l'invention, la phase continue est une phase aqueuse et la composition se présente sous forme d'émulsions H/E. Les compositions à phase continue ou externe aqueuse présentent l'avantage de s'étaler facilement, d'être légères, de bien pénétrer dans la peau, d'être non collantes et d'apporter de la fraîcheur à la peau lors de l'application, contrairement à des compositions à phase continue huileuse.

### Phase aqueuse

Avantageusement, la phase aqueuse contient de l'eau et éventuellement un ou plusieurs composés miscibles à l'eau ou au moins en partie miscibles à l'eau, comme les polyols ; les mono-alcools inférieurs en C₂ à C₈, tels que l'éthanol et l'isopropanol ; et les cétones en C₃ à C₄ liquides à température ambiante. Par "température ambiante", il faut comprendre une température d'environ 25°C, à pression atmosphérique normale (760 mm de Hg).

Par "polyol", il faut comprendre toute molécule organique comportant au moins deux groupements hydroxyle libres. Comme polyols, on peut citer par exemple la glycérine, les glycols comme le butylène glycol, le propylène glycol, l'isoprène glycol, et les polyéthylène glycols comme le PEG-8, le sorbitol, les sucres comme le glucose.

La phase aqueuse peut comprendre aussi tout additif habituel hydrosoluble ou hydrodispersible comme indiqué ci-après.

La phase aqueuse peut représenter de 10 à 95 % en poids, de préférence de 20 à 90 % en poids, mieux de 30 à 80 % en poids, et encore mieux de 40 à 70 % en poids par rapport au poids total de la composition.

Le ou les composés miscibles à l'eau, tels que polyols et alcools inférieurs, peuvent être présents en une quantité allant de 0 à 30 % du poids total de la composition notamment de 0,1 à 30 % et mieux en une quantité allant de 1 à 20 %.

### Phase huileuse

La phase huileuse est une phase grasse liquide à température ambiante (25°C). Elle contient au moins un corps gras choisi parmi les huiles liquides à température ambiante (20-25°C), volatiles ou non, les gommes et les corps gras pâteux, d'origine animale, végétale, minérale ou synthétique, et leurs mélanges. Ces corps gras sont physiologiquement acceptables.

La phase huileuse peut comprendre aussi tout additif habituel liposoluble ou lipodispersible comme indiqué ci-après.

La phase huileuse contient au moins une huile. On entend par "huile" un corps gras liquide à la température ambiante (25°C).

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de coriandre, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrytyle ;
- les hydrocarbures linéaires ou ramifiés, d'origine minérale ou synthétique, tels que les huiles de paraffine, volatiles ou non, et leurs dérivés, la vaseline, les polydécènes, l'isohexadecane, l'isododecane, le polyisobutène hydrogéné tel que l'huile de Parléam® ;
- des alcools gras ayant de 8 à 26 atomes de carbone, comme l'alcool cétylique, l'alcool stéarylique et leur mélange (alcool cétéarylique), l'octyl dodécanol, le 2-butyloctanol, le 2-hexyldécanol, le 2-undécylpentadécanol ou l'alcool oléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclopentasiloxane et la cyclohexadiméthylsiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

Selon un mode préféré de réalisation, la composition de l'invention comporte au moins une huile choisie parmi les huiles de silicone, les esters d'acides gras et leurs mélanges. Selon un mode plus particulièrement préféré, la composition de l'invention comporte au moins une huile de silicone volatile, notamment un cyclopolydiméthylsiloxane.

Les autres corps gras pouvant être présents dans la phase huileuse sont par exemple les acides gras comportant de 8 à 30 atomes de carbone, comme l'acide stéarique, l'acide laurique, l'acide palmitique ; les gommes telles que les gommes de silicone (diméthiconol) ; les résines de silicone telles que la trifluorométhyl-C1-4-alkyldimethicone et la Trifluoropropyldimethicone, et les élastomères de silicone comme les produits commercialisés sous les dénominations "KSG" par la société Shin-Etsu, sous la dénomination "Trefil" par la société Dow Corning ou sous les dénominations "Gransil" par la société Grant Industries, et leurs mélanges.

Ces corps gras peuvent être choisis de manière variée par l'homme du métier afin de préparer une composition ayant les propriétés, par exemple de consistance ou de texture, souhaitées.

Selon un mode préféré de réalisation de l'invention, la composition est exempte de cire.

La quantité de phase huileuse dans la composition de l'invention peut aller de 5 à 90 % en poids, de préférence de 10 à 80 % en poids, mieux de 20 à 70 % en poids et encore mieux de 30 à 60 % en poids par rapport au poids total de la composition. La quantité d'huile(s) est de préférence d'au moins 5 % en poids par rapport au poids total de la composition et de préférence d'au moins 8% en poids par rapport au poids total de la composition.

### Emulsionnant

Les émulsions de l'invention contiennent généralement au moins un émulsionnant choisi parmi les émulsionnants amphotères, anioniques, cationiques ou non ioniques, utilisés seuls ou en mélange, et éventuellement un co-émulsionnant. Les émulsionnants sont choisis de manière appropriée suivant l'émulsion à obtenir (E/H ou H/E). Le ou les émulsionnants sont de préférence choisis parmi les émulsionnants non ioniques.

L'émulsionnant et le co-émulsionnant sont généralement présents dans la composition, en une proportion en matière active pouvant aller par exemple de 0,05 à 30 % en poids, de préférence de 0,2 à 20 % en poids et mieux de 0,5 à 10 % en poids par rapport au poids total de la composition.

Pour les émulsions E/H, on peut citer par exemple comme émulsionnants, les dimethicone copolyols tels que le mélange de cyclomethicone et de dimethicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning et le Cetyl dimethicone copolyol vendu sous la dénomination ABIL EM 90^{R} par la société Goldschmidt, ou le mélange polyglycéryl-4 isostéarate/cétyl diméthicone copolyol/hexyllaurate vendu sous la dénomination ABIL WE 09 par la société Goldschmidt. On peut y ajouter aussi un ou plusieurs co-émulsionnants. De manière avantageuse, le co-émulsionnant peut être choisi dans le groupe comprenant les esters alkylés de polyol. Comme esters alkylés de polyol, on peut citer notamment les esters de glycérol et/ou de sorbitan et par exemple l'isostéarate de polyglycérol, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

On peut aussi utiliser comme émulsionnant d'émulsions E/H, un organopolysiloxane solide élastomère réticulé comportant au moins un groupement oxyalkyléné, tel que ceux obtenus selon le mode opératoire des exemples 3, 4 et 8 du document US-A-5,412,004 et des exemples du document US-A-5,811,487, notamment le produit de l'exemple 3 (exemple de synthèse) du brevet US-A-5,412,004. et tel que celui commercialisé sous la référence KSG 21 par la société Shin Etsu.

Pour les émulsions H/E, on peut citer par exemple comme émulsionnants, les tensioactifs non ioniques, et notamment les esters de polyols et d'acide gras à chaîne saturée ou insaturée comportant par exemple de 8 à 24 atomes de carbone et mieux de 12 à 22 atomes de carbone, et leurs dérivés oxyalkylénés, c'est-à-dire comportant des unités oxyéthylénés et/ou oxypropylénés, tels les esters de glycéryle et d'acide gras en C₈-C₂₄, et leurs dérivés oxyalkylénés ; les esters de polyéthylène glycol et d'acide gras en C₈-C₂₄, et leurs dérivés oxyalkylénés ; les esters de sorbitol et d'acide gras en C₈-C₂₄, et leurs dérivés oxyalkylénés ; les esters de sucre (sucrose, glucose, alkylglucose) et d'acide gras en C₈-C₂₄, et leurs dérivés oxyalkylénés ; les éthers d'alcools gras ; les éthers de sucre et d'alcools gras en C₈-C₂₄, et leurs mélanges.

Comme ester de sorbitol et d'acide gras oxyalkylénés, on peut citer notamment l'ester de sorbitan et d'acide isostéarique à 20 groupes oxyéthylénés (nom INCI : PEG-20 Sorbitan Isostearate) tel que le produit commercialisé sous la dénomination Nikkol TI 10 V par la société Nikkol.

Comme esters de glycéryle et d'acide gras, on peut citer notamment le stéarate de glycéryle (mono-, di- et/ou tri-stéarate de glycéryle) (nom INCI : glyceryl stearate) ou le ricinoléate de glycéryle, et leurs mélanges.

Comme esters de polyéthylène glycol et d'acide gras, on peut citer notamment le stéarate de polyéthylène glycol (mono-, di- et/ou tri-stéarate de polyéthylène glycol), et plus spécialement le monostéarate de polyéthylène glycol 50 OE (nom INCI : PEG-50 stearate), le monostéarate de polyéthylène glycol 100 OE (nom INCI : PEG-100 stearate) et leurs mélanges.

On peut aussi utiliser des mélanges de ces tensioactifs, comme par exemple le produit contenant du Glyceryl stearate et du PEG-100 stearate, commercialisé sous la dénomination ARLACEL 165 par la société Uniqema, et le produit contenant du Glyceryl stearate (mono-distéarate de glycéryle) et du stéarate de potassium, commercialisé sous la dénomination TEGIN par la société Goldschmidt (nom INCI : glyceryl stearate SE).

Comme esters d'acide gras et de glucose ou d'alkylglucose, on peut citer en particulier le palmitate de glucose, les sesquistéarates d'alkylglucose comme le sesquistéarate de méthylglucose, les palmitates d'alkylglucose comme le palmitate de méthylglucose ou d'éthylglucose, les esters gras de méthylglucoside et plus spécialement le diester de méthylglucoside et d'acide oléique (nom INCI : Methyl glucose dioleate) ; l'ester mixte de méthylglucoside et du mélange acide oléique / acide hydroxystéarique (nom INCI : Methyl glucose dioleate/hydroxystearate) ; l'ester de méthylglucoside et d'acide isostéarique (nom INCI : Methyl glucose isostearate) ; l'ester de méthylglucoside et d'acide laurique (nom INCI : Methyl glucose laurate) ; le mélange de monoester et de diester de méthylglucoside et d'acide isostéarique (nom INCI : Methyl glucose sesqui-isostearate) ; le mélange de monoester et de diester de méthylglucoside et d'acide stéarique (nom INCI : Methyl glucose sesquistearate) et en particulier le produit commercialisé sous la dénomination Glucate SS par la société AMERCHOL, et leurs mélanges.

Comme éthers oxyéthylénés d'acide gras et de glucose ou d'alkylglucose, on peut citer par exemple les éthers oxyéthylénés d'acide gras et de méthylglucose, et en particulier l'éther de polyéthylène glycol de diester de méthyl glucose et d'acide stéarique à environ 20 moles d'oxyde d'éthylène (nom INCI : PEG-20 methyl glucose distearate) tel que le produit commercialisé sous la dénomination Glucam E-20 distearate par la société AMERCHOL ; l'éther de polyéthylène glycol du mélange de monoester et de diester de méthyl glucose et d'acide stéarique à environ 20 moles d'oxyde d'éthylène (nom INCI : PEG-20 methyl glucose sesquistearate) et en particulier le produit commercialisé sous la dénomination Glucamate SSE-20 par la société AMERCHOL et celui commercialisé sous la dénomination Grillocose PSE-20 par la société GOLDSCHMIDT, et leurs mélanges.

Comme esters de sucrose, on peut citer par exemple le palmito-stéarate de saccharose, le stéarate de saccharose, le monolaurate de saccharose et leurs mélanges.

Comme éthers d'alcools gras, on peut citer par exemple les éthers dé polyéthylène glycol et d'alcool gras comportant de 8 à 30 atomes de carbone, et notamment de 10 à22 atomes de carbone, tels que les éthers de polyéthylène glycol et d'alcools cétylique, stéarylique, cetéarylique (mélange d'alcools cétylique et stéarylique). On peut citer par exemple les éthers comportant de 1 à 200 et de préférence de 2 à 100 groupes oxyéthylénés, tels que ceux de nom INCI Ceteareth-20, Ceteareth-30, et leurs mélanges.

Comme éthers de sucre, on peut citer notamment les alkylpolyglucosides, et par exemple le decylglucoside comme le produit commercialisé sous la dénomination MYDOL 10 par la société Kao Chemicals, le produit commercialisé sous la dénomination PLANTAREN 2000 par la société Henkel, et le produit commercialisé sous la dénomination ORAMIX NS 10 par la société Seppic ; le caprylyl/capryl glucoside comme le produit commercialisé sous la dénomination ORAMIX CG 110 par la Société Seppic ou sous la dénomination LUTENSOL GD 70 par la Société BASF; le laurylglucoside comme les produits commercialisés sous les dénominations PLANTAREN 1200 N et PLANTACARE 1200 par la société Henkel ; le coco-glucoside comme le produit commercialisé sous la dénomination PLANTACARE 818/UP par la société Henkel ; le cétostéaryl glucoside éventuellement en mélange avec l'alcool cétostéarylique, commercialisé par exemple sous la dénomination MONTANOV 68 par la société Seppic, sous la dénomination TEGO-CARE CG90 par la société Goldschmidt et sous la dénomination EMULGADE KE3302 par la société Henkel, ainsi que l'arachidyl glucoside, par exemple sous la forme du mélange d'alcools arachidique et béhénique et d'arachidyl glucoside commercialisé sous la dénomination MONTANOV 202 par la société Seppic. et leurs mélanges

Selon un mode préféré de réalisation de l'invention, la composition est une émulsion H/E, et l'émulsionnant est choisi parmi les esters de sorbitol et d'acide gras oxyalkylénés, et en particulier l'émulsionnant est l'ester de sorbitan et d'acide isostéarique à 20 groupes oxyéthylénés ; ces émulsionnants permettent d'obtenir une composition à la fois lisse, auto-cicatrisante et très stable, ayant en outre de très bonnes propriétés cosmétiques.

### Additifs

De façon connue, la composition cosmétique ou dermatologique de l'invention peut contenir également un ou plusieurs des adjuvants habituels dans le domaine cosmétique ou dermatologique. Comme adjuvants, on peut citer par les gélifiants, les actifs, les conservateurs, les antioxydants, les parfums, les solvants, les charges, les filtres solaires (= filtres U.V.), les matières colorantes, les agents basiques (triéthanolamine, diéthanolamine, hydroxyde de sodium) ou acides (acide citrique), et encore les vésicules lipidiques ou tout autre type de vecteur (nanocapsules, microcapsules, etc...), et leurs mélanges. Ces adjuvants sont utilisés dans les proportions habituelles dans le domaine cosmétique, et par exemple de 0,01 à 30 % du poids total de la composition, et ils sont, selon leur nature, introduits dans la phase aqueuse de la composition ou dans la phase huileuse, ou encore dans des vésicules ou tout autre type de vecteur. Ces adjuvants ainsi que leurs concentrations doivent être tels qu'ils ne modifient pas la propriété recherchée pour l'émulsion de l'invention.

Selon la fluidité de la composition que l'on souhaite obtenir, on peut incorporer dans la composition, un ou plusieurs gélifiants, notamment hydrophiles, c'est-à-dire solubles ou dispersibles dans l'eau. Comme gélifiants hydrophiles, on peut citer par exemple les polymères carboxyvinyliques modifiés ou non, tels que les produits commercialisés sous les dénominations Carbopol (nom INCI : carbomer) et Pemulen (nom INCI : Acrylates/C10-30 akyl acrylate crosspolymer) par la société Goodrich ; les polyacrylamides ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Hoechst sous la dénomination « Hostacerin AMPS » (nom INCI : ammonium polyacryldimethyltauramide) ; les copolymères anioniques réticulés d'acrylamide et d'AMPS, se présentant sous la forme d'une émulsion E/H, tels ceux commercialisés sous le nom de SEPIGEL 305 (nom C.T.F.A. : Polyacrylamide / C13-14 Isoparaffin / Laureth-7) et sous le nom de SIMULGEL 600 (nom C.T.F.A. : Acrylamide / Sodium acryloyldimethyltaurate copolymer / Isohexadecane / Polysorbate 80) par la société SEPPIC ; les biopolymères polysaccharidiques comme la gomme de xanthane, la gomme de guar, les alginates, les celluloses modifiées ou non ; et leurs mélanges.

Comme charges qui peuvent être utilisées dans la composition de l'invention, on peut citer par exemple, les pigments tels que les oxydes de titane, de zinc ou de fer et les pigments organiques ; le kaolin ; la silice ; le talc ; le nitrure de bore ; les poudres sphérique organiques, et leurs mélanges. Comme poudres sphériques organiques, on peut citer par exemple les poudres de polyamide et notamment les poudres de Nylon® telles que Nylon-1 ou Polyamide 12, vendues sous les dénominations ORGASOL par la société Atochem ; les poudres de polyéthylène ; le Téflon® ; les microsphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; les microsphères de polyméthacrylate de méthyle, commercialisées sous la dénomination MICROSPHERE M-100 par la société Matsumoto ou sous la dénomination COVABEAD LH85 par la société Wackherr ; les poudres de copolymère éthylène-acrylate, comme celles commercialisées sous la dénomination FLOBEADS par la société Sumitomo Seika Chemicals ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ; et leurs mélanges. Ces charges peuvent être présentes dans des quantités allant de 0 à 20 % en poids et de préférence de 1 à 10 % en poids par rapport au poids total de la composition.

Comme actifs utilisables dans la composition de l'invention, on peut citer par exemple, les enzymes (par exemple lactoperoxydase, lipase, protéase, phospholipase, cellulases) ; les flavonoïdes ; les agents hydratants tels que les hydrolysats de protéines ; le hyaluronate de sodium ; les polyols comme la glycérine, les glycols comme les polyéthylène glycols, et les dérivés de sucre ; les anti-inflammatoires ; les oligomères procyannidoliques ; les vitamines comme la vitamine A (rétinol), la vitamine E (tocophérol), la vitamine C (acide ascorbique), la vitamine B5 (panthénol), la vitamine B3 (niacinamide), les dérivés de ces vitamines (notamment esters) et leurs mélanges ; l'urée ; la caféine ; les dépigmentants tels que l'acide kojique, l'hydroquinone et l'acide caféique ; l'acide salicylique et ses dérivés ; les alpha-hydroxyacides tels que l'acide lactique et l'acide glycolique et leurs dérivés ; les rétinoïdes tels que les caroténoïdes et les dérivés de vitamine A ; l'hydrocortisone ; la mélatonine ; les extraits d'algues, de champignons, de végétaux, de levures, de bactéries ; les stéroïdes ; les actifs anti-bactériens comme le 2,4,4'-trichloro-2'-hydroxy diphényl éther (ou triclosan), le 3,4,4'-trichlorocarbanilide (ou triclocarban) et les acides indiqués ci-dessus et notamment l'acide salicylique et ses dérivés ; les agents tenseurs ; les céramides ; les huiles essentielles ; et leurs mélanges ; et tout actif approprié pour le but final de la composition.

Comme exemples de stéroïdes, on peut citer la déhydroépiandrostérone (ou DHEA), ainsi que (1) ses précurseurs et dérivés biologiques, en particulier les sels et esters de DHEA, tels que le sulfate et le salicylate de DHEA, la 7-hydroxy DHEA, la 7-céto DHEA, les esters de 7-hydroxy et 7-céto DHEA, notamment la 3-beta-acétoxy-7-oxo DHEA, et (2) ses précurseurs et dérivés chimiques, en particulier les sapogénines telles que la diosgénine ou l'hécogénine, et/ou leurs dérivés tels que l'acétate d'hécogénine, et/ou les extraits naturels en contenant et notamment les extraits de Dioscorées, tels que l'igname sauvage (Wild Yam).

Les filtres U.V. peuvent être organiques ou minéraux (ou filtres U.V. physiques)

Les filtres U.V. peuvent être présents en une quantité en matière active allant de 0,01 à 20 % en poids de matière active, de préférence de 0,1 à 15 % en poids, et mieux 0,2 à 10 % en poids par rapport au poids total de la composition.

Comme exemples de filtres organiques, actifs dans l'UV-A et/ou l'UV-B, pouvant être ajoutés dans la composition de l'invention, on peut citer par exemple, les dérivés à fonction sulfonique tels que les dérivés sulfonés ou sulfonatés du benzylidène camphre, de la benzophénone ou du phénylbenzimidazole, plus particulièrement les dérivés du benzylidène camphre, comme l'acide benzène 1,4 [di(3-méthylidène-campho 10-sulfonique)], (nom INCI : Terephthalylidene Dicamphor Sulfonic Acid) fabriqué sous le nom « MEXORYL SX » par la société CHIMEX. l'acide 4'-sulfo 3-benzylidènecamphre (nom INCI : Benzylidene Camphor Sulfonic Acid), fabriqué sous le nom « MEXORYL SL» par la société CHIMEX, l'acide 2-[4-(camphométhylidène) phényl] benzimidazole-5-sulfonique, l'acide phénylbenzimidazole sutfonique (nom INCI : Phenylbenzimidazole Sulfonic Acid), commercialisé sous le nom EUSOLEX 232 par la société MERCK ; les dérivés de l'acide para-aminobenzoique ; les dérivés salicyliques tels que le salicylate d'éthyl hexyle vendu sous le nom commercial NEO HELIOPAN OS par Haarmann et Reimer ; les dérivés du dibenzoylméthane tels que le Butyl Methoxydibenzoylmethane vendu notamment sous le nom commercial PARSOL 1789 par Hoffmann La Roche ; les dérivés cinnamiques tels que l'éthylhexyl Methoxycinnamate vendu notamment sous le nom commercial PARSOL MCX par Hoffmann La Roche ; les dérivés de β,β'-diphénylacrylate tels que l'octocrylene (α-cyano-β,β-diphénylacrylate de 2-éthylhexyle) vendu sous le nom commercial UVINUL N539 par la société BASF ; les dérivés de la benzophénone tels que la Benzophenone-1 vendu sous le nom commercial UVINUL 400 par BASF, la Benzophenone-2 vendu sous le nom commercial UVINUL D50 par BASF, la Benzophenone-3 ou Oxybenzone, vendu sous le nom commercial UVINUL M40 par BASF, la Benzophenone-4 vendu sous le nom commercial UVINUL MS40 par BASF ; les Dérivés du benzylidène camphre tels que le 4-Methylbenzylidene camphre vendu sous le nom commercial EUSOLEX 6300 par MERCK ; les dérivés du phenyl benzimidazole tels que le Benzimidazilate vendu sous le nom commercial NEO HELIOPAN AP par Haarmann et Reimer ; les dérivés de la triazine tels que l'Anisotriazine vendu sous le nom commercial TINOSORB S par CIBA GEIGY et l'éthylhexyl triazone vendu notamment sous le nom commercial UVINUL T150 par BASF ; les dérivés du phenyl benzotriazole tels que Drometrizole Trisiloxane vendu sous le nom commercial SILATRIZOLE par Rhodia Chimie ; les dérivés anthraniliques tels que le Menthyl anthranilate vendu sous le nom commercial NEO HELIOPAN MA par Haarmann et Reimer ; les dérivés d'imidazolines ; les dérivés du benzalmalonate ; et leurs mélanges.

Comme filtres physiques pouvant être ajoutés dans la composition de l'invention, on peut citer par exemple les pigments et nano-pigments d'oxydes métalliques, enrobés ou non enrobés, notamment les oxydes de titane, de fer, de zirconium, de zinc ou de cérium, et leurs mélanges, ces oxydes pouvant être sous forme de micro- ou nanoparticules (nanopigments), éventuellement enrobées.

La composition de l'invention est destinée à une application topique et peut constituer notamment une composition dermatologique ou cosmétique, par exemple destinée au soin, au traitement, au nettoyage et au maquillage des matières kératiniques et notamment de la peau, des lèvres, des cheveux, des cils et des ongles des êtres humains.

Ainsi, elle peut constituer une composition de traitement ou de soin de la peau (y compris le cuir chevelu), des fibres kératiniques (cheveux, cils, sourcils), des ongles ou des lèvres, ou une composition de protection solaire ou de bronzage artificiel, ou encore un produit nettoyant ou démaquillant de la peau, des cheveux, des sourcils ou des cils, un produit déodorant ou encore un produit parfumant. Elle est alors généralement non colorée ou faiblement colorée, et elle peut contenir éventuellement des actifs cosmétiques ou dermatologiques. Elle peut alors être utilisée comme base de soin pour la peau ou les lèvres (baumes à lèvres, protégeant les lèvres du froid et/ou du soleil et/ou du vent), comme crème de soin de jour ou de nuit pour la peau du visage et/ou du corps.

La composition selon l'invention peut également constituer une composition cosmétique colorée et notamment un produit de maquillage de la peau, en particulier un fond de teint, un blush, un fard à joues ou à paupières, un mascara, un eye-liner, présentant éventuellement des propriétés de soin ou de traitement, un tatouage corps.

Selon un mode préféré de réalisation de l'invention, la composition est destinée à une application topique sur la peau.

Ainsi, l'invention a encore pour objet l'utilisation cosmétique d'une composition cosmétique telle que définie ci-dessus, pour le soin, le traitement, le nettoyage et/ou le maquillage des matières kératiniques, de préférence la peau.

Elle a aussi pour objet un procédé cosmétique de soin ou de traitement des matières kératiniques et notamment de la peau, des cheveux ou des lèvres des êtres humains, comprenant l'application sur ces matières kératiniques d'une composition en particulier cosmétique telle que définie ci-dessus. La matière kératinique est de préférence la peau.

La composition selon l'invention est préparée selon les méthodes usuelles. dans les domaines d'application considérés.

L'invention est illustrée plus en détail dans l'exemple suivant. Les composés sont indiqués selon les cas en nom INCI (= CTFA) ou en nom chimique, et les pourcentages sont donnés en poids sauf mention contraire.

### Exemple 1 : Crème blanche (Emulsion H/E)

*Phase A*
   - Cyclohexasiloxane 23 %
   - Alcool cétylique 0,5 %
   - Alcool stéarylique 0,5 %
   - PEG-20 Sorbitan Isostearate (Nikkol TI V 10) 1,25 %
*Phase B*
   - Glycérine 5 %
   - Conservateurs qsp 1 %
   - Eau qsp 100 %
*Phase C*
   - Steareth-100/PEG-136/HMDI Copolymer (SER-AD FX1100) 2,24 %
   - Eau 20,16 %
*Phase D*
   - Fibres de polyamide (Nylon 66)
      (Polyamide 0.9 dtex, 0,3 mm - Société Paul Bonte) 6 %
   - Aluminum Starch Octenylsuccinate (Dry Flo) 3 %

Mode opératoire : On mélange sous agitation les constituants de la phase A et la phase B séparément à chaud (environ 70 à 80°C), et on introduit la phase huileuse (phase A) dans la phase aqueuse (phase B), sous agitation. On refroidit le mélange jusqu'à environ 40°C, puis on y introduit la phase C préalablement préparée sous agitation. On incorpore en dernier la phase D.

On obtient une crème blanche, dans laquelle les fibres sont réparties de façon homogène et qui est très douce à l'application. Cette crème peut être utilisée par exemple pour le soin de la peau. Le dépôt obtenu est bien homogène et reste homogène au cours du temps, et on n'observe pas d'aspect filandreux dans la composition au cours de la durée d'utilisation.

### Exemple 2 : Crème blanche (Emulsion H/E)

*Phase A*
   - Cyclohexasiloxane 15 %
   - Methyl Glucose Sesquistearate 2 %
   - Triglycéride caprylique/caprique (Mygliol 812) 10 %
*Phase B*
   - Glycérine 7 %
   - Conservateurs 1 %
   - Eau qsp 100 %
*Phase C*
   - PEG-20 Methyl Glucose Sesquistearate 3 %
   - Eau 19 %
*Phase D*
   - Fibres de polyamide (Nylon 66)
      (Polyamide 0.9 dtex, 0,3 mm - Société Paul Bonte) 8 %
*Phase E*
   - Steareth-100/PEG-136/HMDI Copolymer (SER-AD FX1100) 2,5 %
   - Eau 20,16 %
*Phase F*
   - Kaolin 2 %

Mode opératoire : On mélange sous agitation les constituants de la phase A et la phase B séparément à chaud (environ 70 à 80°C), et on introduit la phase huileuse (phase A) dans la phase aqueuse (phase B), sous agitation. On refroidit le mélange jusqu'à environ 40°C, puis on y introduit la phase C préalablement préparée sous agitation. On incorpore en dernier les phases D, E et F.

On obtient une crème blanche, dans laquelle les fibres sont réparties de façon homogène et qui est douce à l'application. Cette crème peut être utilisée par exemple pour le soin de la peau. Le dépôt obtenu est bien homogène et reste homogène au cours du temps, et on n'observe pas d'aspect filandreux dans la composition au cours de l'utilisation.

## Revendications

1. Composition pour application topique comprenant dans un milieu physiologiquement acceptable, des fibres et au moins un polyuréthanne associatif, le milieu comprenant une phase aqueuse et une phase huileuse comprenant au moins une huile.

2. Composition selon la revendication précédente, **caractérisée en ce que** les fibres sont choisies parmi les fibres de soie, de coton, de laine, de lin, de cellulose extraites notamment du bois, des légumes ou des algues, de polyamide, de rayonne, de viscose, d'acétate, de poly-p-phénylène téréphtalamide, en acrylique, de polyoléfine, de verre, de silice, d'aramide, de carbone notamment sous forme graphite, de Téflon®, de collagène insoluble, de polyesters, de polychlorure de vinyle ou de vinylidène, d'alcool polyvinylique, de polyacrylonitrile, de chitosane, de polyuréthane, de polyéthylène phtalate, des fibres de mélanges de polymères, des fibres chirurgicales, et leurs mélanges.

3. Composition selon la revendication 1 ou 2, **caractérisée en ce que** les fibres sont des fibres d'origine synthétique.

4. Composition selon l'une quelconque des revendications précédentes, **caractérisée en ce que** les fibres sont des fibres de polyamide ou de poly-p-phénylène téréphtalamide.

5. Composition selon l'une quelconque des revendications précédentes, **caractérisée en ce que** les fibres ont une longueur allant de 0,1 à 1,5 mm.

6. Composition selon l'une quelconque des revendications précédentes, **caractérisée en ce que** les fibres ont un diamètre moyen allant de 5 à 50 µm.

7. Composition selon l'une quelconque des revendications précédentes, **caractérisée en ce que** les fibres sont choisies parmi les fibres de Nylon 6, de Nylon 6,6, et leurs mélanges.

8. Composition selon l'une quelconque des revendications précédentes, **caractérisée en ce que** les fibres sont présentes en une quantité allant de 0,5 à 50 % en poids par rapport au poids total de la composition.

9. Composition selon l'une des revendications précédentes, **caractérisée en ce que** la quantité de polyuréthanne associatif va de 0,5 à 10 % en poids par rapport au poids total de la composition.

10. Composition selon l'une des revendications précédentes, **caractérisée en ce que** le polyuréthanne associatif est un copolymère comportant au moins deux chaînes lipophiles hydrocarbonées ayant de 6 à 30 atomes de carbone, séparées par une séquence hydrophile.

11. Composition selon la revendication précédente, **caractérisée en ce que** la séquence hydrophile est une chaîne oxyéthylénée.

12. Composition selon l'une des revendications précédentes, **caractérisée en ce que** le polyuréthanne associatif est un polymère tribloc.

13. Composition selon l'une des revendications précédentes, **caractérisée en ce que** le polyuréthanne associatif est soluble dans l'eau.

14. Composition selon l'une quelconque des revendications précédentes, **caractérisée en ce que** la phase aqueuse représente de 10 à 95 % en poids par rapport au poids total de la composition.

15. Composition selon l'une quelconque des revendications précédentes, **caractérisée en ce que** la phase huileuse contient au moins une huile choisie parmi les huiles de silicone, les esters d'acides gras et leurs mélanges.

16. Composition selon l'une quelconque des revendications précédentes, **caractérisée en ce que** la quantité d'huile(s) est d'au moins 5 % en poids par rapport au poids total de la composition.

17. Composition selon l'une quelconque des revendications précédentes, **caractérisée en ce qu'**elle contient en outre au moins un émulsionnant.

18. Composition selon l'une quelconque des revendications précédentes, **caractérisée en ce qu'**elle se présente sous forme d'une émulsion huile-dans-eau.

19. Composition selon la revendication précédente, **caractérisée en ce qu'**elle contient en outre au moins un émulsionnant choisi parmi les esters de sorbitol et d'acide gras oxyalkylénés.

20. Composition selon l'une quelconque des revendications précédentes, **caractérisée en ce qu'**elle constitue une composition dermatologique ou cosmétique.

21. Utilisation cosmétique d'une composition cosmétique selon l'une quelconque des revendications 1 à 19, pour le soin, le traitement, le nettoyage et/ou le maquillage des matières kératiniques, de préférence la peau.

22. Procédé cosmétique de soin ou de traitement des matières kératiniques des êtres humains, comprenant l'application sur ces matières kératiniques d'une composition cosmétique selon l'une quelconque des revendications 1 à 19.

23. Procédé selon la revendication précédente, **caractérisée en ce que** la matière kératinique est la peau.

24. Utilisation cosmétique d'un polyuréthanne associatif, dans une composition pour application topique, contenant des fibres, pour obtenir une composition constamment lisse et homogène.
